(19) Europäisches Patentamt
European Patent Office
Office européen des brevets

(11) Publication number: **0 188 225 B1**

(12) # EUROPEAN PATENT SPECIFICATION

(45) Date of publication of the patent specification: **07.01.88**

(51) Int. Cl.[4]: **C 07 C 157/14**, C 07 D 249/14, C 07 D 409/12 // C07D487/04

(21) Application number: **86100188.1**

(22) Date of filing: **08.01.86**

(54) **Process for the preparation of herbicidal sulfonamides derived from substituted 2-amino-1,2,4 triazolo (1,5-a) pyrimidines and novel intermediates provided therein.**

(30) Priority: **14.01.85 US 691331**

(43) Date of publication of application: **23.07.86 Bulletin 86/30**

(45) Publication of the grant of the patent: **07.01.88 Bulletin 88/1**

(84) Designated Contracting States: **BE DE FR GB IT NL**

(56) References cited:
**EP - A - 0 057 564**
**GB - A - 1 064 141**

(73) Proprietor: **THE DOW CHEMICAL COMPANY, 2030 Dow Center Abbott Road P.O. Box 1967, Midland, MI 48640 (US)**

(72) Inventor: **Kleschick, William A., 640 Fig Tree Lane, Martinez California 94553 (US)**
Inventor: **Vinogradoff, Anna P., 1135-D Kenwal Road, Concord California 94521 (US)**
Inventor: **Dunbar, Joseph E., 5813 Sturgeon, Creek Parkway Midland Michigan 48640 (US)**

(74) Representative: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 188 225 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Compounds of general structure I

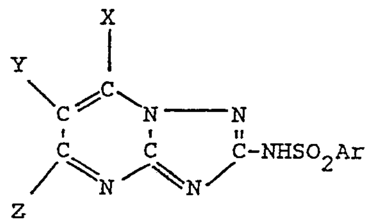

(I)

wherein Ar represents an aromatic (substituted or unsubstituted) or heteroaromatic (substituted or unsubstituted) ring system exhibit herbicidal activity against a variety of weed species.

While some of the compounds in this family may be prepared by a conventional, convergent approach *via* the requisite substituted 2-amino-1,2,4-triazolo[1,5-a]pyrimidine, many analogs cannot be made following this approach. The highly insoluble nature of many of the appropriate substituted 2-amino-1,2,4-triazolo[1,5-a]pyrimidines prohibits their reaction with the less reactive and less stable aromatic sulfonyl chlorides.

We have found a new method for the preparation of compounds of type I which eliminate most of the limitations of methodology which had been previously described. The method of this invention also provides novel intermediates as described more fully hereinafter.

The novel procedure for the preparation of compounds of formula I is illustrated in Scheme I. The starting aromatic sulfonyl chlorides II and sulfonamide III are commercially available or may be prepared from appropriate starting materials as known in the art. In accordance with this invention the sulfonamides III are reacted with dimethyl N-cyanodithioiminocarbonate in the presence of a base in a solvent. Bases which are effective in this transformation include tertiary amine (i.e. triethylamine) or alkali metal hydroxides, alkoxides or carbonates (i.e. NaOH, $NaOCH_3$ or $K_2CO_3$). Appropriate solvents include acetone, methyl ethyl ketone acetonitrile or tetrahydrofuran (THF). The reaction may be run at temperatures ranging from ambient temperature to reflux. The products of this transformation (IV) may be isolated directly as their salts and converted to their neutral species by acidification. In some instances the salt may be used directly in subsequent transformations without purification or conversion of the corresponding neutral species. Compound IV may be reacted with an excess of hydrazine to form the intermediate 1,2,4-triazoles V. This reaction is generally carried out in solvents such as acetonitrile, THF, DMF, or DMSO at ambient temperature although higher temperatures may be employed to increase the rate of reaction. The amount of excess hydrazine utilized in this transformation ranges from 5 to 400 mole percent. The final step in this sequence for the conversion of compound V to I may be carried out as generally outlined in "Heterocyclic Systems with Bridgehead Nitrogen Atoms", Part Two, W. L. Mosby, Interscience Publishers, 1961, p. 878. A wide variety of 1,3-dicarbonyl compounds may be used in this reaction which may be run under acidic (i.e. acetic acid as a solvent), neutral (i.e. DMF as a solvent) or basic conditions (i.e. using alkali metal alkoxides or carbonates in polar aprotic solvents such as DMF or DMSO).

Scheme I

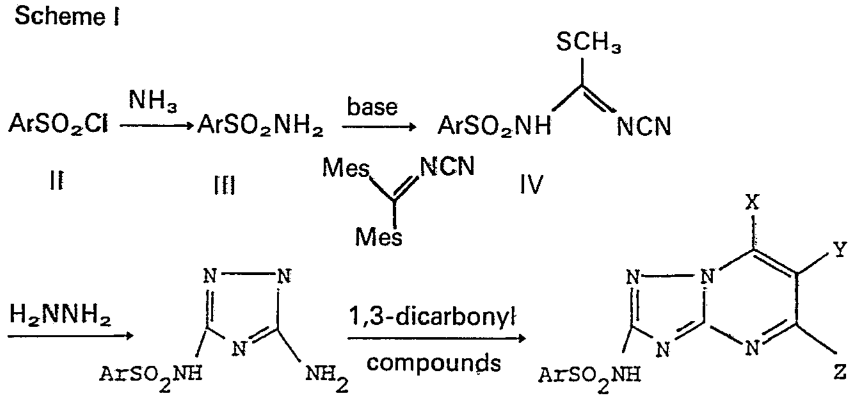


II III IV V I

In examples where unsymmetrical 1,3-dicarbonyl compounds are employed in the process outlined in Scheme I, the possibility of obtaining two different isomeric condensation products exists. Often the appropriate choice of reaction conditions (i.e. acidic or basic) allows for control of the regiochemistry of the annulation process.

In cases where β-ketoesters or malonic esters are used in the last step of the process described above, the products contain hydroxy groups (i.e. I where X and/or Z is OH). These products may be subjected to further transformations involving conversion of the hydroxy groups to chlorine with phosphorous oxychloride. The resulting halo substituted compounds are capable of undergoing reaction with nucleophiles to affect nucleophilic substitution of the halogen. This procedure is highly useful in the preparation of alkoxy, alkylthio and amino substituted heterocyclic ring systems.

An alternative procedure for the synthesis of intermediates of general structure IV is illustrated in equation 1. The starting materials (VI) may be prepared from aromatic sulfonamides by known art (i.e. F. L. Merchan, *Synthesis*, 994 (1982); R. Gompper, et al., *Chem. Ber.*, 99, 2885, 2990 (1966)). These intermediates may be reacted with cyanamide in the presence of base. Bases include tertiary amines and alkali metal alkoxides, hydroxides and carbonates. This reaction is most frequently carried out in acetonitrile or THF at temperatures ranging from ambient temperature to reflux.

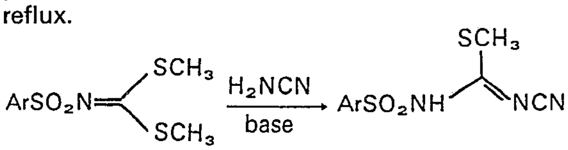


VI IV

In certain instances intermediates VI may convert to their corresponding mono or dichloro derivatives (i.e. VII and VIII respectively). This may be accomplished by known art (i.e. E. Kuhle, et al., *Angew, Chem Int. Ed. Engl.*, *6*, 649 (1967)). These intermediates may then be advantageously used in a manner analogous to VI in the synthesis of compounds of general structure I.

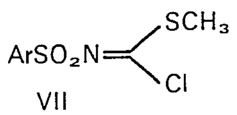

VII

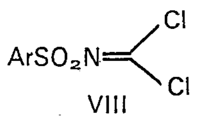

VIII

Compounds advantageously prepared by the process of this invention have the general formula:

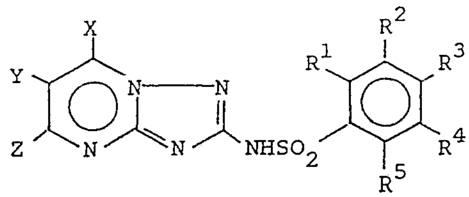

wherein $R^1$ represents halo (F, Cl, Br, I), $-NO_2$, phenyl, OAr, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$ and $-SO_3CH_2CF_3$; $R^2$ and $R^4$ represent H, halo (F, Cl, Br, I), $C_1$-$C_4$ alkyl, $COOR^7$ and $-OR^8$; $R^3$ is H; and $R^5$ represents H, $C_1$ to $C_4$ alkyl, halo (F, Cl, Br, I), $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$, $-SO_3CH_2CF_3$, $-CR^6R^6OR^6$ and $-CR^6R^6SR^6$ wherein Ar represents substituted or unsubstituted aryl or substituted or unsubstituted heteroaryl, $R^6$ represents H, aryl or $C_1$-$C_4$ alkyl, $R^7$ represents $C_1$-$C_6$ alkyl, alkenyl, alkynyl, aryl, substituted alkyl or substituted aryl and $R^8$ and $R^9$ individually represent $C_1$-$C_4$ alkyl; and X, Y and Z represent H, $C_1$-$C_4$ alkyl, $C_1$-$C_4$ alkoxy halo (F, Cl, Br, I), or X and Y or Y and Z can be joined to form a cycloalkyl ring (i.e., $-(CH_2)_n-$ wherein n is 3 or 4) or X and Y or Y and Z can be joined to form a ring containing a heteroatom (i.e., $-O(CH_2)_n-$ wherein n is 2 or 3). In this specification aryl and heteroaryl refer to 5- or 6-membered aromatic or heteroaromatic ring systems wherein the substituents are, individually and preferably, hydrogen, halogen, nitro, $C_1$-$C_4$ alkyl, $C_1$-$C_4$ mono- or polyhaloalkyl, $C_1$-$C_4$ alkoxy, $C_1$-$C_4$ mono- or polyhaloalkoxy or amino.

The following examples illustrate the invention.

*Example 1*

*N'-Cyano-N-(2-nitrophenylsulfonyl)-S-methylisothiourea*

A mixture of 2.02 g (10.0 mmol) of 2-nitrobenzene-sulfonamide, 1.46 g (10.0 mmol) of dimethyl N-cyanodithioiminocarbonate and 1.38 g (10.0 mmol) of powdered, anhydrous $K_2CO_3$ in 16 ml of acetone was heated at reflux for 20 hours. The reaction mixture was filtered and the solid collected was washed several times with acetone. The filtrate was evaporated and the orange oily residue was triturated with ether to afford a solid. The solid was collected by filtration, washed with ether and suspended in 10 ml of $^1N$ HCl. After stirring for 1 hour the solid was collected by filtration, washed with water and dried to yield 1.65 g (55 percent) of the desired product as a cream colored solid, mp 122° C (decomposition). IR and $^1H$ NMR spectra were consistent with the assigned structure.

*Analysis* for $C_9H_8N_4O_4S_2$:

| | | | | |
|---|---|---|---|---|
| Calculated: | C 36.00 | H 2.69 | N 18.66 | S 21.35% |
| Found: | C 36.10 | H 2.74 | N 18.72 | S 21.22% |

*Example 2*

*N-(5-Amino-1,2,4-triazol-3-yl)-2-nitrobenzenesulfonamide*

A suspension of 29.4 g (98.0 mmol) of N'-Cyano-N-(2-nitrophenylsulfonyl)-S-methylisothiourea in 100 ml of acetonitrile was treated with 6.2 ml (6.3 g, 0.20 mol) of anhydrous hydrazine. A mild exothermic reaction occurred as the reaction mixture became homogeneous. After stirring for 9 days the precipitated solid was collected by filtration and dried to afford 22.9 g of yellow solid. The crude product was recrystallized from HOAc to yield a total of 15.9 g (57 percent) of the desired product as a pale yellow solid, mp 255-256° C. IR and $^1H$ NMR spectra were consistent with the assigned structure.

*Analysis* for $C_8H_8N_6O_4S$:

| | | | | |
|---|---|---|---|---|
| Calculated: | C 33.80 | H 2.84 | N 29.57 | S 11.28% |
| Found: | C 34.11 | H 2.79 | N 29.35 | S 11.50% |

*Example 3*

*N-(5,7-dimethyl-1,2,4-triazolo[1,5-a]pyrimidin-2-yl)-2-nitrobenzenesulfonamide*

A mixture of 2.43 g (9.00 mmol) of N-(5-amino-1,2,4-triazol-3-yl)-2-nitrobenzenesulfonamide and 1.85 ml (1.80 g. 18.0 mmol) of 2,4 pentanedione in 25 ml of glacial acetic acid was heated at reflux for 19 hours. After cooling to room

temperature, the solid which separated was collected by filtration, washed with acetic acid and dried *in vacuo* to yield 2.58 g (82 percent) of the desired product as an off-white crystalline solid, mp 255-256° C. IR and $^1$H NMR spectra were in agreement with the assigned structure.

*Analysis* for $C_{13}H_{12}N_6O_4S$:

| | | | | |
|---|---|---|---|---|
| Calculated: | C 44.83 | H 3.47 | N 24.14 | S 9.20% |
| Found: | C 44.88 | H 3.34 | N 24.51 | S 9.09% |

*Example 4*

*N'-Cyano-N-(2,5-dichlorophenylsulfonyl)-S-methylisothiourea*

A solution of 10.6 g (43.2 mmol) of 2.5-dichlorobenzenesulfonamide, 7.15 g (44.0 mmol) of 90 percent dimethyl N-cyanodithioiminocarbonate and 1.8 g (44 mmol) of NaOH in 60 ml of ethanol and 10 ml of $H_2O$ was heated at reflux for 6 hours. After cooling to room temperature the reaction mixture was poured into 600 ml of ice water. The resulting solution was acidified with 6N HCl to separate 2.2 g of the desired product as a white solid. Concentration of the filtrate gave an additional 8.5 g of the desired product. The total yield of material was 10.7 g (76 percent) of white solid, mp 145° C. IR and $^1$H NMR spectra were consistent with the assigned structure.

*Analysis* for $C_9H_7Cl_2N_3O_2S_2$:

| | | | |
|---|---|---|---|
| Calculated: | C 33.34 | H 2.18 | N 12.96% |
| Found: | C 33.50 | H 2.39 | N 12.82% |

*Example 5*

*N-(5-Amino-1,2,5-triazol-3-yl)-2,5-dichlorobenzenesulfonamide*

A mixture of 8.51 g (26.2 mmol) of N'-cyano-N-(2,5-dichlorophenylsulfonyl)-S-methylisothiourea and 10 ml (10 g, 0.20 mol) of hydrazine monohydrate in 85 ml of ethanol was heated at reflux for 30 minutes. After cooling to room temperature, the solid which separated was collected and suspended in 170 ml of $H_2O$ and the suspension was acidified with concentrated aqueous HCl. After stirring the suspension for 4 hours the solid was collected and dried *in vacuo* to yield 5.10 g (57 percent) of the desired product as a hydrochloride salt, mp 306-308° C. IR and $^1$H NMR spectra were consistent with the assigned structure.

*Analysis* for $C_8H_7Cl_2N_5O_2S \cdot HCl$:

| | | | |
|---|---|---|---|
| Calculated: | C 27.88 | H 2.34 | N 20.32% |
| Found: | C 28.36 | H 2.50 | N 19.78% |

*Example 6*

*N-(5,7-dimethyl-1,2,4-triazolo[1,5-a]pyrimidin-2-yl)-2,5-dichlorobenzenesulfonamide*

A solution of 4.60 g (13.3 mmol) of N-(5-amino-1,2,4-triazol-3-yl)-2,5-dichlorobenzenesulfonamide hydrochloride and 4.0 g (40 mmol) of 2,4-pentanedione in 60 ml of glacial acetic acid was heated at reflux for 4 hours. The reaction mixture was cooled to room temperature and poured into 500 ml of ice water to separate a solid. The solid was collected by filtration and dried to yield 4.53 g (92 percent) of the desired product as a white solid, m.p. 216.5°-218.5° C.

*Analysis* for $C_{13}H_{11}Cl_2N_5O_2S$:

| | | | |
|---|---|---|---|
| Calculated: | C 41.95 | H 2.98 | N 18.81% |
| Found: | C 41.83 | H 3.10 | N 18.67% |

*Example 7*

*2-Chloro-N-(5-methyl-7-trifluoromethyl-1,2,4-triazolo[1,5-a]pyrimidin-2yl)benzensulfonamide*

A mixture of 2.19 g (8.00 mmol) of N-(5-amino-1,2,4-triazol-3-yl)-2-chlorobenzenesulfonamide and 1.09 ml (1.38 g, 8.96 mmol) of 1,1,1-trifluoro-2,4-pentanedione in 9 ml of glacial acetic acid was heated at reflux for 21 hours. After cooling to room temperature, the reaction mixture was poured into a mixture of ice and water. The solid which separated was collected by filtration, washed with water and dried to yield 2.90 g (93 percent) of the desired product as a white solid, mp 203-204.5° C. IR and $^1$H NMR spectra were in agreement with the assigned structure.

*Analysis* for $C_{13}H_9ClF_3N_5O_2S$:

| | | | | |
|---|---|---|---|---|
| Calculated: | C 39.86 | H 2.32 | N 17.88 | Cl 9.05 S 8.18% |
| Found: | C 40.23 | H 2.31 | N 18.22 | Cl 9.13 S 8.26% |

*Example 8*

*2-Chloro-N-(7-methyl-1,2,4-triazolo-[1,5-a]pyrimidin-2-yl)benzenesulfonamide*

A sample of 3.0 ml (2.7 g, 20 mmol) of acetylacetaldehyde dimethylacetal was added to a solution of 2.74 g (10.0 mmol) of N-(5-amino-1,2,4-triazol-3-yl)-2-chlorobenzenesulfonamide in 20 ml of glacial acetic acid at reflux over 12 hours. After the addition was complete the reaction mixture was heated at reflux for 15 hours and cooled to room temperature. The solid which separated was collected by filtration, washed with acetic acid and dried to yield 1.92 g (59 percent) of the desired product as white solid, mp 267.5-269° C. IR and $^1$H NMR spectra were in agreement with the assigned structure.

*Analysis* for $C_{12}H_{10}ClN_5O_2S$:

| | | | | |
|---|---|---|---|---|
| Calculated: | C 44.52 | H 3.11 | N 21.63 | Cl 10.95 S 9.90% |
| Found: | C 44.36 | H 3.07 | N 21.69 | Cl 10.82 S 10.15% |

*Example 9*

*2-Chloro-N-(1,2,4-triazolo[1,5-a]pyrimidin-2-yl)benzenesulfonamide*

A mixture of 2.74 g (10.0 mmol) of N-(5-amino-1,2,4-triazol-3-yl)-2-chlorobenzenesulfonamide and 3.3 ml (3.3 g, 20 mmol) of malonaldehyde bis(dimethylacetal) in 10 ml of glacial acetic acid was heated at reflux for 24 hours. After cooling to room temperature, the solid which separated was collected by filtration, washed with acetic acid and dried to yield 1.78 g (58 percent)

of the desired product as a tan solid, mp 253.5-256.5° C. IR and $^1$H NMR spectra were in agreement with the assigned structure.

*Analysis* for $C_{11}H_8ClN_5O_2S$:

| | |
|---|---|
| Calculated: | C 42.66 H 2.60 N 22.61 Cl 11.45 S 10.35% |
| Found: | C 42.97 H 2.60 N 22.42 Cl 11.19 S 10.07% |

*Example 10*

*2-Chloro-N-(6-chloro-1,2,4-triazolo[1,5-a]pyrimidin-2-yl)benzenesulfonamide*

A mixture of 2.46 g (9.00 mmol) of N-(5-amino-1,2,4-triazol-3-yl)-2-chlorobenzenesulfonamide and 1.67 g (9.90 mmol) of mucochloric acid in 20 ml of DMF was heated to reflux for 16.5 hours. After cooling to room temperature, the solvent was removed by evaporation at reduced pressure and the residue was treated with 20 ml of 0.5N NaOH. After stirring vigorously for ~ 30 minutes the mixture was filtered through celite and the filtrate was acidified with 2N HCl. The solid which separated was collected by filtration, washed with water and recrystallized from acetic acid - water to yield 0.70 g (23 percent) of the desired product as a light brown solid, mp 256.5-260.5° C. IR and $^1$H NMR spectra were in agreement with the assigned structure.

*Analysis* for $C_{11}H_7Cl_2N_5O_2S$:

| | |
|---|---|
| Calculated: | C 38.39 H 2.05 N 20.35 Cl 20.60 S 9.32% |
| Found: | C 38.74 H 2.08 N 20.84 Cl 19.54 S 8.70% |

*Example 11*

*2-Chloro-N-(6-methyl-1,2,4-triazolo-[1,5-a]pyrimidin-2-yl)benzenesulfonamide*

A mixture of 2.02 g (8.36 mmol) of 1,3-bis-(dimethylamino)-2-methyltrimethinium perchlorate and 2.29 g (8.36 mmol) of N-(5-amino-1,2,4-triazol-3-yl)-2-chlorobenzenesulfonamide in 25 ml of glacial acetic acid was heated at reflux for 19 hours. The solvent was removed by evaporation at reduced pressure, and the residue was treated with 20 ml of 0.5N NaOH. Some additional [1]N NaOH was added to dissolve all of the material (~ pH 10). The solution was filtered and the filtrate was acidified with 2N HCl to precipitate a solid. The solid was collected by filtration, washed with water and dried to yield 2.34 g (87 percent) of the desired product as a pale yellow solid, mp 236-239° C. IR and $^1$H NMR spectra were in agreement with the assigned structure.

*Analysis* for $C_{12}H_{10}ClN_5O_2S$:

| | |
|---|---|
| Calculated: | C 44.52 H 3.11 N 21.63 Cl 10.95 S 9.90% |
| Found: | C 44.17 H 3.05 N 21.93 Cl 11.01 S 9.69% |

*Example 12*

*N-(5-Amino-1,2,4-triazol-3-yl)-2,6-dichlorobenzenesulfonamide*

A mixture of 90.1 g (0.398 mol) of 2,6-dichlorobenzenesulfonamide, 64.7 g (0.398 mol) of dimethyl N-cyanodithioiminocarbonate and 58.3 g (0.420 mol) of powdered anhydrous $K_2CO_3$ in 800 ml of THF was heated at reflux for 3 hours. After cooling to 30° C, 25.3 ml (25.6 g, 0.798 mol) of anhydrous hydrazine was added dropwise over 30 minutes. The resulting mixture was stirred for 3 days at ambient temperature and filtered. The solid collected was washed with THF, suspended in 400 ml of water and acidified with 180 ml of acetic acid. The resulting mixture was filtered, and the solid collected was washed with water and dried to yield 112 g (91 percent) of the desired product as a white solid, mp 300° C (decomp.). IR and $^1$H NMR spectra were in agreement with the assigned structure.

*Analysis* for $C_8H_7Cl_2N_5O_2S$:

| | |
|---|---|
| Calculated: | C 31.18 H 2.29 N 22.73 Cl 23.01 S 10.40% |
| Found: | C 31.39 H 2.26 N 22.70 Cl 22.88 S 10.25% |

Following the above general procedure and employing appropriate starting materials the compounds indicated in the tables were prepared.

*Table I*

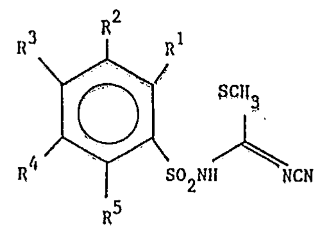

| R¹ | R² | R³ | R⁴ | R⁵ | Physical Properties | Elemental Composition | C | H | N | Cl | S |
|---|---|---|---|---|---|---|---|---|---|---|---|
| H | H | H | H | H | mp 122°C (decomp.) | Exact mass calcd. for $C_9H_9N_3O_2S_2$: 255.0136<br>Found: 255.0125 | | | | | |
| $NO_2$ | H | H | H | H | mp 122°C (decomp.) | *Anal.* Calcd. for $C_9H_8N_4O_4S_2$:<br>Found: | 36.00<br>36.10 | 2.69<br>2.74 | 18.66<br>12.95 | —<br>— | 21.35<br>21.22 |
| $CF_3$ | H | H | H | H | mp 128°C (decomp.) | *Anal.* Caldc. for $C_{10}H_8F_3N_3O_2S_2$:<br>Found: | 37.15<br>37.02 | 2.49<br>2.55 | 13.00<br>12.95 | —<br>— | 19.83<br>20.00 |
| Cl | H | H | H | H | mp 126.5°C (decomp.) | *Anal.* Calcd. for $C_9H_8ClN_3O_2S_2$:<br>Found: | 37.31<br>37.15 | 2.78<br>2.82 | 14.50<br>14.67 | 12.24<br>12.40 | 22.13<br>21.90 |
| $CH_3$ | H | H | H | H | mp 129°C (decomp.) | Exact mass calcd. for $C_{10}H_{11}N_3O_2S_2$: 269.0292<br>Found: 269.0299 | | | | | |
| H | H | $CH_3$ | H | H | mp 137.5-139°C (decomp.) | *Anal.* Calcd. for $C_{10}H_{11}N_3O_2S_2$:<br>Found: | 44.59<br>44.80 | 4.12<br>4.16 | 15.60<br>15.53 | —<br>— | —<br>— |
| H | H | Cl | H | H | mp 140-140.5°C (decomp.) | *Anal.* Calcd. for $C_9H_8ClN_3O_2S$:<br>Found: | 37.31<br>37.50 | 2.78<br>2.97 | 14.50<br>14.68 | —<br>— | —<br>— |
| H | H | $OCH_3$ | H | H | mp 141.5°C | *Anal.* Calcd. for $C_{10}H_{11}N_3O_3S_2$:<br>Found: | 42.09<br>42.14 | 3.89<br>4.02 | 14.73<br>14.86 | —<br>— | —<br>— |
| *i*-Pr | H | *i*-Pr | H | *i*-Pr | mp 165-165.5°C | *Anal.* Calcd. for $C_{18}H_{27}N_3O_2S_2$:<br>Found: | 56.66<br>56.90 | 7.13<br>7.15 | 11.01<br>10.97 | —<br>— | —<br>— |
| Cl | H | H | H | Cl | mp 239-241°cC | *Anal.* Calcd. for $C_9H_7Cl_2N_3O_2S_2$:<br>Found: | 33.34<br>33.14 | 2.18<br>2.12 | 12.96<br>12.91 | 21.87<br>21.73 | 19.78<br>19.62 |

*Table II*

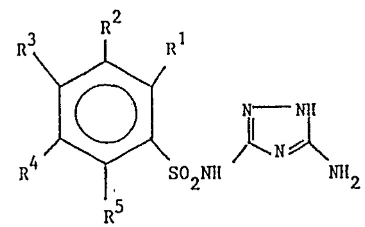

| $R^1$ | $R^2$ | $R^3$ | $R^4$ | $R^5$ | Physical Properties | Elemental Composition | C | H | N | Cl | S |
|---|---|---|---|---|---|---|---|---|---|---|---|
| H | H | H | H | H | mp 293°C-294°C | *Anal.* Calcd. for $C_8H_9N_5O_2S$:<br>Found: | 40.16<br>40.29 | 3.79<br>3.67 | 29.27<br>29.40 | —<br>— | 13.40<br>13.26 |
| $CF_3$ | H | H | H | H | mp 281°C-238°C (decomp.) | *Anal.* Calcd. for $C_9H_8F_3N_5O_2S$:<br>Found: | 35.18<br>35.01 | 2.62<br>2.57 | 22.79<br>22.91 | —<br>— | 10.44<br>10.22 |
| $NO_2$ | H | H | H | H | mp 255-256°C (decomp.) | *Anal.* Caldc. for $C_8H_8N_6O_4S$:<br>Found: | 33.80<br>34.11 | 2.84<br>2.79 | 29.57<br>29.35 | —<br>— | 11.28<br>11.50 |
| Cl | H | H | H | H | mp 307-309°C (decomp.) | *Anal.* Calcd. for $C_8H_8ClN_5O_2S$:<br>Found: | 35.11<br>34.84 | 2.95<br>2.90 | 25.59<br>25.56 | 12.95<br>13.03 | 11.71<br>11.56 |
| $CH_3$ | H | H | H | H | mp 285-286°C | *Anal.* Calcd. for $C_9H_{11}N_5O_2S$:<br>Found: | 42.68<br>42.59 | 4.38<br>4.17 | 27.65<br>27.79 | —<br>— | 12.66<br>12.46 |
| H | H | $CH_3$ | H | H | mp 314-315°C | *Anal.* Calcd. for $C_9H_{11}N_5O_2S$:<br>Found: | 42.68<br>42.34 | 4.38<br>4.34 | 27.65<br>27.44 | —<br>— | —<br>— |
| H | H | Cl | H | H | mp 314-315°C (decomp.) | *Anal.* Calcd. for $C_8H_8ClN_5O_2S$:<br>Found: | 35.11<br>34.90 | 2.95<br>3.12 | 25.59<br>25.30 | —<br>— | —<br>— |
| H | H | $OCH_3$ | H | H | mp 300°C (decomp.) | *Anal.* Calcd. for $C_9H_{11}N_5O_3S$:<br>Found: | 40.14<br>40.10 | 4.12<br>4.25 | 26.01<br>25.72 | —<br>— | —<br>— |
| *i*-Pr | H | *i*-Pr | H | *i*-Pr | mp 314.5°C (decomp.) | *Anal.* Calcd. for $C_{17}H_{27}N_5O_2S$:<br>Found: | 55.86<br>56.17 | 7.45<br>7.49 | 19.16<br>19.18 | —<br>— | —<br>— |
| Cl | H | H | H | Cl | mp 295-296°C (decomp.) | *Anal.* Calcd. for $C_8H_7Cl_2N_5O_2S$:<br>Found: | 31.18<br>31.35 | 2.29<br>2.20 | 22.73<br>22.70 | 23.01<br>23.05 | 10.40<br>10.26 |
| F | H | H | H | F | mp 322-323°C (decomp.) | *Anal.* Calcd. for $C_8H_7N_5O_2S$:<br>Found: | 34.91<br>34.93 | 2.56<br>2.53 | 25.45<br>25.72 | —<br>— | —<br>— |
| Cl | Cl | H | H | H | mp 326-327°C (decomp.) | *Anal.* Calcd. for $C_8H_7Cl_2N_5O_2S$:<br>Found: | 31.18<br>31.17 | 2.29<br>2.24 | 22.73<br>23.22 | 23.01<br>22.30 | 10.40<br>9.80 |

*Table III*

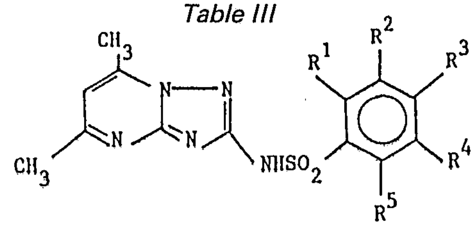

| $R^1$ | $R^2$ | $R^3$ | $R^4$ | $R^5$ | Physical Properties | Elemental Composition | C | H | N | Cl | S |
|---|---|---|---|---|---|---|---|---|---|---|---|
| $CF_3$ | H | H | H | H | mp 246.5-248°C | *Anal.* Calcd. for $C_{14}H_{12}F_3N_5O_2S$:<br>Found: | 45.28<br>45.31 | 3.26<br>3.33 | 18.86<br>18.74 | —<br>— | 8.63<br>8.71 |
| $CF_3$ | H | H | H | H | mp 202.5-203.5°C | *Anal.* Calcd. for $C_{14}H_{15}N_5O_2S$:<br>Found: | 52.98<br>52.43 | 4.76<br>4.56 | 22.07<br>21.78 | —<br>— | 10.10<br>9.59 |
| $NO_2$ | H | H | H | H | mp 255-256°C (decomp.) | *Anal.* Caldc. for $C_{13}H_{12}N_6O_4S$:<br>Found: | 44.83<br>44.88 | 3.47<br>3.34 | 24.13<br>24.51 | —<br>— | 9.20<br>9.09 |
| Cl | H | H | H | H | mp 216.5-219.5°C | *Anal.* Calcd. for $C_{13}H_{12}ClN_5O_2S$:<br>Found: | 46.23<br>45.97 | 3.58<br>3.66 | 20.73<br>21.01 | 10.50<br>10.73 | 9.49<br>9.30 |
| H | H | $CH_3$ | H | H | mp 241-241.5°C | *Anal.* Calcd. for $C_{14}H_{15}N_5O_2S$:<br>Found: | 52.98<br>52.70 | 4.76<br>4.96 | 22.07<br>22.02 | —<br>— | —<br>— |
| H | H | Cl | H | H | mp 254-255.5°C | *Anal.* Calcd. for $C_{13}H_{12}ClN_5O_2S$:<br>Found: | 42.22<br>46.10 | 3.58<br>3.75 | 20.73<br>20.69 | —<br>— | —<br>— |
| H | H | $OCH_3$ | H | H | mp 198.5-199.5°C | *Anal.* Calcd. for $C_{14}H_{15}N_5O_3S$:<br>Found: | 50.44<br>50.43 | 4.54<br>4.70 | 21.01<br>20.99 | —<br>— | —<br>— |
| *i*-Pr | H | *i*-Pr | H | *i*-Pr | mp 283°C (decomp.) | *Anal.* Calcd. for $C_{22}H_{31}N_5O_2S$:<br>Found: | 61.51<br>61.30 | 7.27<br>7.33 | 16.30<br>16.28 | —<br>— | —<br>— |
| Cl | H | H | H | Cl | mp 259-261°C | *Anal.* Calcd. for $C_{13}H_{11}Cl_2N_5O_2S$:<br>Found: | 41.95<br>41.86 | 2.98<br>2.94 | 18.81<br>19.09 | 19.05<br>18.92 | 8.61<br>8.39 |
| F | H | H | H | F | mp 261-262°C | *Anal.* Calcd. for $C_{13}H_{11}F_2N_5O_2S$:<br>Found: | 46.02<br>45.94 | 3.27<br>3.19 | 20.64<br>20.79 | —<br>— | —<br>— |
| Cl | Cl | H | H | H | mp 231-233°C | *Anal.* Calcd. for $C_{11}H_7Cl_2N_5O_2S$:<br>Found: | 41.95<br>41.83 | 2.98<br>2.90 | 18.81<br>19.55 | —<br>— | —<br>— |

*Table IV*

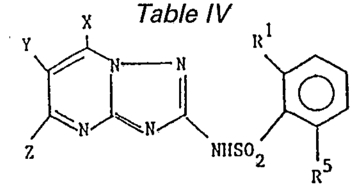

| R¹ | R⁵ | X | Y | Z | Physical Properties | Elemental Composition | C | H | N | Cl | S |
|---|---|---|---|---|---|---|---|---|---|---|---|
| Cl | H | H | Cl | H | mp 258.5-260.5°C | *Anal.* Calcd. for $C_{11}H_7Cl_2N_5O_2S$:<br>Found: | 38.39<br>38.74 | 2.05<br>2.08 | 20.35<br>20.84 | 20.60<br>19.54 | 9.32<br>8.70 |
| Cl | H | $CH_3$ | Cl | $CH_3$ | mp 266-269°C | *Anal.* Calcd. for $C_{13}H_{11}Cl_2N_5O_2S$:<br>Found: | 41.95<br>42.51 | 2.98<br>3.03 | 18.81<br>18.98 | 19.05<br>18.16 | 8.61<br>8.41 |
| Cl | H | $CF_3$ | H | $CH_3$ | mp 203-204.5°C | *Anal.* Calcd. for $C_{13}H_9ClF_3N_5O_2S$:<br>Found: | 39.86<br>40.23 | 2.32<br>2.31 | 17.88<br>18.22 | 9.05<br>9.13 | 8.18<br>8.26 |
| Cl | H | $CF_3$ | H | $CF_3$ | mp 224-226°C | *Anal.* Calcd. for $C_{13}H_6ClF_6N_5O_2S$:<br>Found: | 35.02<br>35.31 | 1.36<br>1.38 | 15.71<br>15.95 | 7.95<br>7.72 | 7.19<br>7.40 |
| Cl | H | H | $CH_3$ | H | mp 236-239°C | *Anal.* Calcd. for $C_{12}H_{10}ClN_5O_2S$:<br>Found: | 44.52<br>44.17 | 3.11<br>3.05 | 21.63<br>21.93 | 10.95<br>11.01 | 9.90<br>9.69 |
| Cl | H | $CH_3$ | $CH_3$ | $CH_3$ | mp 273-280°C | *Anal.* Calcd. for $C_{14}H_{14}ClN_5O_2S$:<br>Found: | 47.80<br>47.78 | 4.01<br>3.90 | 19.91<br>20.19 | 10.08<br>10.20 | 9.11<br>9.17 |
| Cl | H | $CH_2CH_3$ | H | $CH_2CH_3$ | mp 216-218°C | *Anal.* Calcd. for $C_{15}H_{16}ClN_5O_2S$:<br>Found: | 49.25<br>49.18 | 4.41<br>4.36 | 19.14<br>19.45 | 9.69<br>9.64 | 8.76<br>8.75 |
| Cl | H | H | H | H | mp 253.5-256.5°C | *Anal.* Calcd. for $C_{11}H_8ClN_5O_2S$:<br>Found: | 42.66<br>42.97 | 2.60<br>2.60 | 22.61<br>22.42 | 11.45<br>11.19 | 10.35<br>10.07 |
| Cl | H | $CH_3$ | H | H | mp 267.5-269°C | *Anal.* Calcd. for $C_{12}H_{10}ClN_5O_2S$:<br>Found: | 44.52<br>44.36 | 3.11<br>3.07 | 21.63<br>21.69 | 10.95<br>10.82 | 9.90<br>10.15 |
| Cl | Cl | $CH_3$ | Cl | $CH_3$ | mp 296-297°C | *Anal.* Calcd. for $C_{13}H_{10}Cl_3N_5O_2S$:<br>Found: | 38.39<br>38.48 | 2.48<br>2.44 | 17.22<br>17.58 | —<br>— | —<br>— |
| Cl | Cl | H | Cl | H | mp 262-264°C | *Anal.* Calcd. for $C_{11}H_6Cl_3N_5O_2S$:<br>Found: | 34.89<br>34.72 | 1.60<br>1.78 | 18.50<br>19.12 | —<br>— | —<br>— |
| Cl | Cl | H | H | H | mp 264-269°C | *Anal.* Calcd. for $C_{11}H_7Cl_2N_5O_2S$:<br>Found: | 38.38<br>38.29 | 2.05<br>2.05 | 20.35<br>20.08 | 20.61<br>19.80 | 9.32<br>9.13 |
| Cl | Cl | $CF_3$ | H | $CF_3$ | mp 238-240°C | *Anal.* Calcd. for $C_{13}H_5F_6N_5O_2S$:<br>Found: | 32.51<br>32.19 | 1.05<br>1.01 | 14.59<br>14.57 | 14.77<br>14.51 | 6.68<br>6.86 |
| Cl | Cl | $CH_3$ | $CH_3$ | $CH_3$ | mp 347°C (decomp.) | *Anal.* Calcd. for $C_{14}H_{13}Cl_2N_5O_2S$:<br>Found: | 43.53<br>43.52 | 3.39<br>3.29 | 18.13<br>18.42 | 18.35<br>18.37 | 8.30<br>8.29 |
| Cl | Cl | $CH_2CH_3$ | H | $CH_2CH_3$ | mp 259-261°C | *Anal.* Calcd. for $C_{15}H_{15}Cl_2N_5O_2S$:<br>Found: | 45.01<br>44.44 | 3.78<br>3.72 | 17.50<br>17.79 | 17.72<br>17.33 | 8.01<br>8.32 |

*Table V*

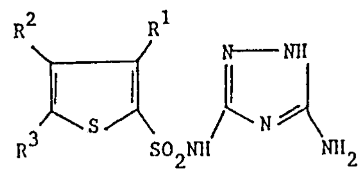

| $R^1$ | $R^2$ | $R^3$ | Physical Properties | Elemental Composition | C | H | N | Cl | S | Br |
|---|---|---|---|---|---|---|---|---|---|---|
| H | H | Cl | mp 310°C (decomp.) | *Anal.* Calcd. for $C_6H_6ClN_5O_2S_2$:<br>Found: | 25.76<br>25.81 | 2.16<br>2.13 | 25.04<br>25.18 | 12.68<br>12.40 | 22.93<br>22.17 | —<br>— |
| H | Br | Br | mp 325°C (decomp.) | *Anal.* Calcd. for $C_6H_5Br_2N_5O_2S_2$:<br>Found: | 17.88<br>18.13 | 1.25<br>1.24 | 17.38<br>17.38 | —<br>— | 15.91<br>15.07 | 39.65<br>37.71 |

*Table VI*

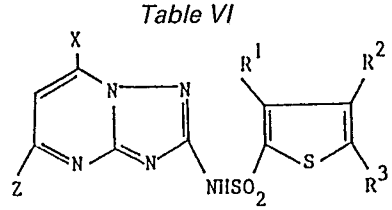

| $R^1$ | $R^2$ | $R^3$ | X | Z | Physical Properties | Elemental Composition | C | H | N | Cl | S | Br |
|---|---|---|---|---|---|---|---|---|---|---|---|---|
| H | H | Cl | $CH_3$ | $CH_3$ | mp 200-202°C | *Anal.* Calcd. for $C_{11}H_{10}ClN_5O_2S_2$:<br>Found: | 38.43<br>38.08 | 2.93<br>2.83 | 20.37<br>20.47 | 10.31<br>8.59 | 18.65<br>18.91 | —<br>— |
| H | Br | Br | $CH_3$ | $CH_3$ | mp 183-185°C | *Anal.* Calcd. for $C_{11}H_9Br_2N_5O_2S_2$:<br>Found: | 28.28<br>28.06 | 1.94<br>1.78 | 14.99<br>14.97 | —<br>— | 13.73<br>13.90 | 34.21<br>33.60 |

**Claims**

1. Process for making a compound having the formula

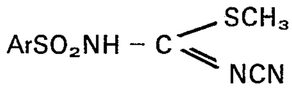

wherein Ar is an aromatic or heteroaromatic, substituted or unsubstituted ring system which comprises reacting a compound having the formula $ArSO_2NH_2$ with dimethyl N-cyanodithioiminocarbonate in the presence of a base in a solvent.

2. Process of Claim 1 wherein the base is a tertiary amine or alkali metal hydroxide or alkoxide or an alkali metal carbonate.

3. Process of Claim 2 wherein the base is sodium hydroxide or potassium carbonate.

4. Process for making a compound having the formula

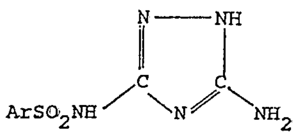

wherein Ar is an aromatic or heteroaromatic substituted or unsubstituted ring system which comprises reacting a compound having the formula

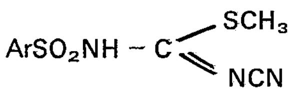

with an excess of hydrazine in the presence of a solvent.

5. Process of Claim 4 wherein the solvent is acetonitrile, tetrahydrofuran, dimethylformamide or dimethylsulfoxide.

6. A compound having the formula

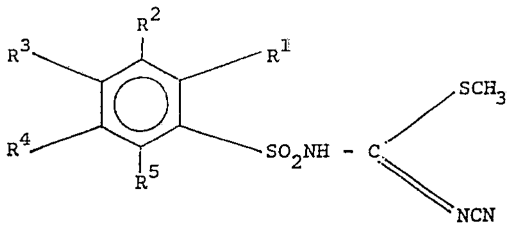

wherein $R^1$ represents halo (F, Cl, Br, I), $-NO_2$, phenyl, OAr, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$ and $-SO_3CH_2CF_3$; $R^2$ and $R^4$ represent H, halo (F, Cl, Br, I), $C_1$-$C_4$ alkyl, $COOR^7$ and $-OR^8$; $R^3$ is H; and $R^5$ represents H, $C_1$ to $C_4$ alkyl, halo (F, Cl, Br, I), $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$, $-SO_3CH_2CF_3$, $-CR^6R^6OR^6$ and $-CR^6R^6SR^6$ wherein Ar represents substituted or unsubstituted aryl or substituted or unsubstituted heteroaryl, $R^6$ represents H, aryl or $C_1$-$C_4$ alkyl, $R^7$ represents $C_1$-$C_6$ alkyl, alkenyl, alkynyl, aryl, substituted alkyl or substituted aryl and $R^8$ and $R^9$ individually represent $C_1$-$C_4$ alkyl.

7. A compound having the formula

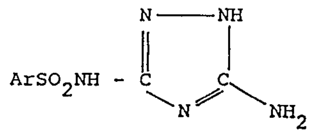

wherein Ar is an aromatic or heteroaromatic substituted or unsubstituted ring system.

8. Compound of Claim 7 wherein Ar is

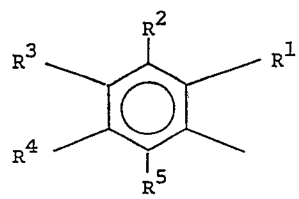

wherein $R^1$, $R^2$, $R^3$, $R^4$ and $R^5$ are as defined in Claim 6.

9. Compound of Claim 7 wherein Ar is

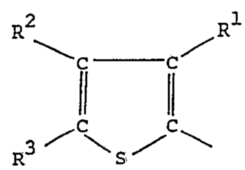

wherein $R^1$, $R^2$ and $R^3$ are as defined in Claim 6.

**Patentansprüche**

1. Verfahren zur Herstellung einer Verbindung mit der allgemeinen Formel:

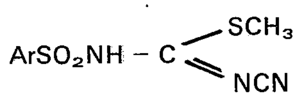

worin Ar ein aromatisches oder heteroaromatisches, substituiertes oder unsubstituiertes Ringsystem ist, das die Umsetzung einer Verbindung mit der Formel $ArSO_2NH_2$ mit Dimethyl-N-cyanodithioiminocarbonat in Anwesenheit einer Base in einem Lösungsmittel umfasst.

2. Verfahren nach Anspruch 1, worin die Base ein tertiäres Amin oder Alkalihydroxid oder Alkoxid oder ein Alkalicarbonat ist.

3. Verfahren nach Anspruch 2, worin die Base Natriumhydroxid oder Kaliumcarbonat ist.

4. Verfahren zur Herstellung einer Verbindung mit der allgemeinen Formel:

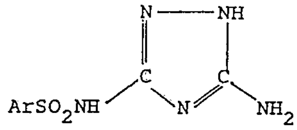

worin Ar ein aromatisches oder heteroaromatisches substituiertes oder unsubstituiertes Ringsystem ist, das die Umsetzung einer Verbindung mit der Formel

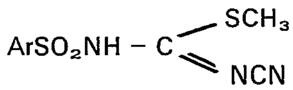

mit einem Überschuss von Hydrazin in Anwesenheit eines Lösungsmittels umfasst.

5. Verfahren nach Anspruch 4, worin das Lösungsmittel Acetonitril, Tetrahydrofuran, Dimethylformamid oder Dimethylsulfoxid ist.

6. Verbindung der allgemeinen Formel:

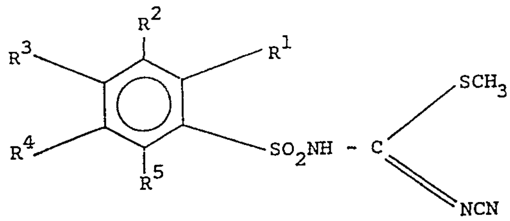

worin $R^1$ ein Halogen (F, Cl, Br, J), $-NO_2$, Phenyl, OAr, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$ und $-SO_3CH_2CF_3$ ist; $R^2$ und $R^4$ H, Halogen (F, Cl, Br, J), $C_1$-$C_4$-Alkyl, $COOR^7$ und $-OR^8$ bedeuten; $R^3$ H bedeutet; und $R^5$ H, $C_1$-$C_4$-Alkyl, Halogen (F, Cl, Br, J), $NO_2$, $CF_3$, $-OCF_3$, $-OCF_2CF_2H$, $-OCF2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$, $-SO_3CH_2CF_3$, $-CR^6R^6OR^6$ und $-CR^6R^6SR^6$ bedeutet, worin Ar eine substituierte oder unsubstituierte Aryl- oder substituierte oder unsubstituierte Heteroarylgruppe bedeutet, $R^6$ H, eine Aryl- oder $C_1$-$C_4$-Alkylgruppe bedeutet, $R^7$ eine $C_1$-$C_6$-Alkyl-, Alkenyl-, Alkynyl-, Aryl-, substituierte Alkyl- oder substituierte Arylgruppe bedeutet und $R^8$ und $R^9$ unabhängig voneinander eine $C_1$-$C_4$-Alkylgruppe bedeuten.

7. Verbindung der allgemeinen Formel:

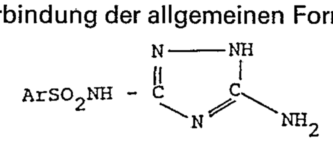

worin Ar ein aromatisches oder heteroaromatisches substituiertes oder unsubstituiertes Ringsystem ist.

8. Verbindung nach Anspruch 7, worin Ar:

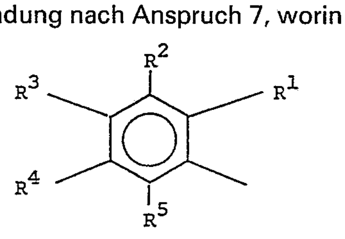

ist, worin $R^1$, $R^2$, $R^3$, $R^4$ und $R^5$ dieselbe Bedeutung wie in Anspruch 6 haben.

9. Verbindung nach Anspruch 7, worin Ar

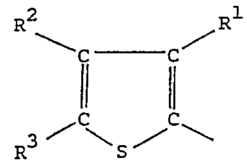

ist, worin $R^1$, $R^2$ und $R^3$ dieselbe Bedeutung wie in Anspruch 6 haben.

## Revendications

1. Procédé de fabrication d'un composé de formule:

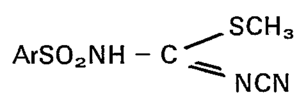

dans laquelle Ar est un système cyclique aromatique ou hétéroaromatique, substitué ou non substitué, qui consiste à faire réagir un composé de formule $ArSO_2NH_2$ avec le N-cyano-di-thiocarbonate de diméthyle, en présence d'une base et dans un solvant.

2. Procédé selon la revendication 1, dans lequel la base est une amine tertiaire, un hydroxyde ou alcoolate de métal alcalin ou un carbonate de métal alcalin.

3. Procédé selon la revendication 2, dans lequel la base est l'hydroxyde de sodium ou le carbonate de potassium.

4. Procédé de préparation d'un composé de formule:

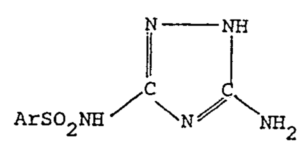

dans laquelle Ar est un système cyclique aromatique ou hétéroaromatique, substitué ou non substitué, qui consiste à faire réagir un composé de formule:

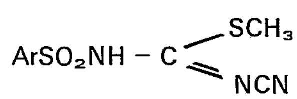

avec un excès d'hydrazine en présence d'un solvant.

5. Procédé selon la revendication 4, dans lequel le solvant est l'acétonitrile, le tétrahydrofuranne, le diméthylformamide ou le diméthylsulfoxyde.

6. Composé répondant à la formule:

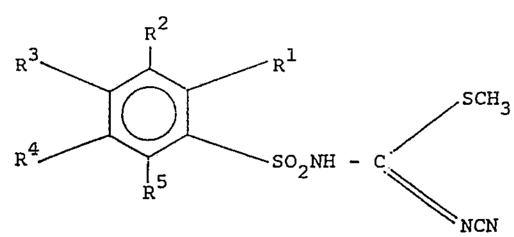

dans laquelle $R^1$ représente un halogène (F, Cl, Br, I), $-NO_2$, un phényle, OAr, $-CF_3$, $-OCF_2CF_2H$, $-OCF_2CCl_2H$, $-OCH_2CF_3$, $-OCF_3$, $-SCF_3$, $-SCF_2CF_2H$, $-SCF_2CCl_2H$, $-SOCF_3$, $-SOCF_2CF_2H$, $-SOCF_2,CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$ ou $-SO_3CH_2CF_3$;$R^2$ et $R^4$ représentent H, un halogène (F, Cl, Br, I), un alkyle en $C_1$-$C_4$, $COOR^7$ ou $-OR^8$; $R^3$ est H; et $R^5$ représente H, un alkyle en $C_1$-$C_4$, un halogène (F, Cl, Br, I), $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONH_2$, $-CONHR^8$, $-CONR^8(R^9)$, $-SO_3R^8$, $-SO_3CH_2CF_3$, $-CR^6R^6OR^6$ ou $-CR^6R^6SR^6$, où Ar représente un aryle substitué ou non substitué ou un hétéroaryle substitué ou non substitué, $R^6$ représente H, un aryle ou un alkyle en $C_1$-$C_4$, $R^7$ représente un groupe alkyle en $C_{1-6}$, alcényle, alkynyle, aryle, alkyle substitué ou aryle substitué, et $R^8$ et $R^9$ représentent individuellement un groupe alkyle en $C_{1-4}$.

7. Composé de formule:

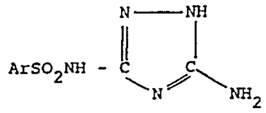

dans laquelle Ar est un système cyclique aromatique ou hétéroaromatique substitué ou non substitué.

8. Composé selon la revendication 7, dans lequel Ar est:

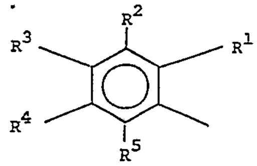

où $R^1$, $R^2$, $R^3$, $R^4$ et $R^5$ sont tels que définis dans la revendication 6.

9. Composé selon la revendication 7, dans lequel Ar est:

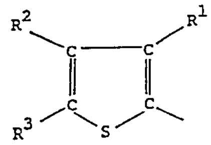

où $R^1$, $R^2$ et $R^3$ sont tels que définis dans la revendication 6.